# EUROPEAN PATENT APPLICATION

(11) **EP 2 732 849 A1**
(43) Date of publication of application: **21.05.2014**
(21) Application number: 12828666.3
(22) Date of filing: 02.04.2012
(51) Int. Cl.: A61N 1/06, A61N 1/30, A61M 5/46, A61B 18/00, A61B 17/34

(54) **COSMETIC DEVICE USING HIGH-FREQUENCY WAVES**

(30) Priority: 26.08.2011 KR 20110085809
(71) Applicant: Amorepacific Corporation, Seoul 140-777 (KR)
(72) Inventor: KIM, Hyuk, Yongin-si Gyeonggi-do 446-729 (KR); KOH, Hyun Ju, Yongin-si Gyeonggi-do 446-729 (KR); PARK, Sun Young, Yongin-si Gyeonggi-do 446-904 (KR); PARK, Won Seok, Yongin-si Gyeonggi-do 446-729 (KR); LEE, Dong Chae, Seoul 133-773 (KR)
(74) Representative: Ahner, Philippe
(86) International application number: PCT/KR2012/002466
(87) International publication number: WO 2013/032094

(57) **Abstract**

The present invention relates to high frequency aesthetic device including: a high frequency transmitting part adapted to transmit the high frequency generated from an external high frequency generating source; and an aesthetic medicine injecting part adapted to apply the high frequency to a patient's skin and inject an aesthetic medicine into the skin, wherein the high frequency transmitting part and the aesthetic medicine injecting part are mechanically separable from each other. According to the present invention, the aesthetic medicine injecting part and the high frequency transmitting part are separable from each other, thereby providing the convenience in use during the surgery, and the degree of insertion of the needle is checkable quantitatively, thereby conducting the surgery in more accurate manner. Additionally, the high frequency aesthetic device is capable of minimizing the damage of the blood vessels during the tunneling through the needle.

## Description

### Technical Field

The present invention relates to a high frequency aesthetic device, and more particularly, to a high frequency aesthetic device that is capable of injecting an aesthetic medicine using high frequency.

### Background Art

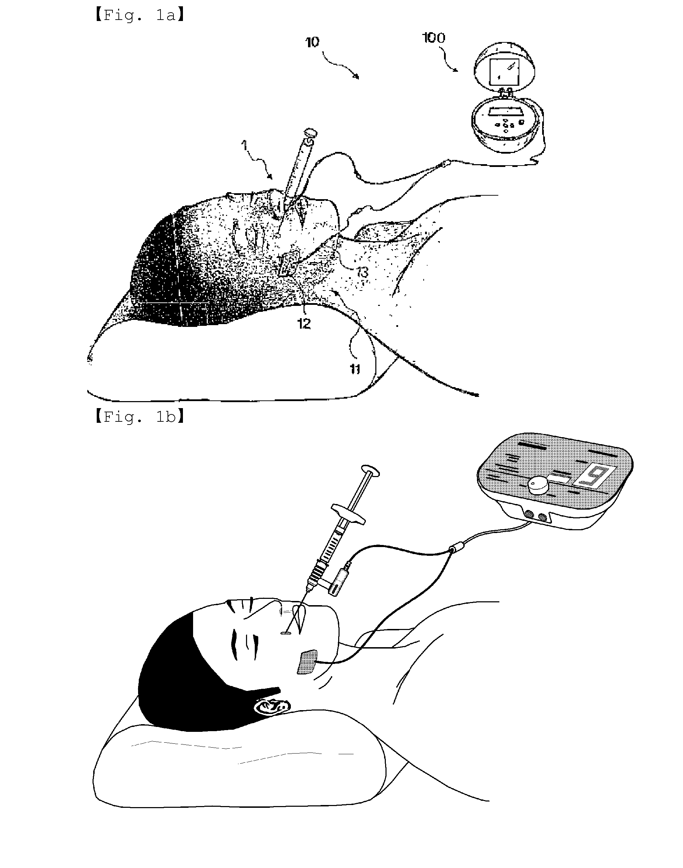

Each of high frequency aesthetic devices in conventional practices is composed of a syringe with a needle mounted thereon and a high frequency electrical signal generator connected to the needle by means of an electric wire. FIGS.1a and 1b are exemplary views showing conventional high frequency aesthetic devices. The conventional high frequency aesthetic device as shown in FIG.1b includes a syringe 1 in which an aesthetic medicine is stored and a high frequency electrical signal generator 100 connected to the syringe 1 by means of an electric wire 13.

According to the conventional high frequency aesthetic devices, as shown in FIGS.1a and 1b, the electric wire 13 for high frequency application is connected directly to the syringe 1. Under the above-mentioned structures of the conventional high frequency aesthetic devices, it is impossible to remove the electric wire 13 from the syringe 1 even after the needle has been inserted into the skin and the high frequency has been applied through the needle, which makes the surgery being conducted after the injection of the aesthetic medicine more complicated. Further, the cost for exchanging consumption goods may be increased. That is, the electric wire should be exchanged at the time when the syringe is exchanged, and contrarily, the syringe should be exchanged at the time when the electric wire is exchanged.

According to the conventional high frequency aesthetic devices, furthermore, it is hard for a surgeon to quantitatively check the degree of insertion of the needle into the skin. That is, the needle of the syringe is inserted into the skin so as to perform the application of the high frequency and the injection of the aesthetic medicine, but the conventional high frequency aesthetic devices do not have any means capable of allowing the degree of insertion of the needle into the skin to be checked quantitatively through the surgeon. In case where the surgery is conducted with the conventional high frequency aesthetic device, the degree of insertion of the needle is recognized just through the sensation or experiences of the surgeon.

Therefore, there is a definite need for a new high frequency aesthetic device which is convenient in use and allows the degree of insertion of the needle to be checked quantitatively.

### Disclosure of the Invention

### Technical Problem

Accordingly, the present invention has been made in view of the above-mentioned problems occurring in the prior art, and it is an object of the present invention to provide a high frequency aesthetic device that is capable of separating an aesthetic medicine injecting part and a high frequency transmitting part from each other and quantitatively checking the degree of insertion of a needle.

It is another object of the present invention to provide a high frequency aesthetic device that is capable of minimizing the damage of the blood vessels during the tunneling through a needle.

### Solution to Problem

To accomplish the above objects, according to an aspect of the present invention, there is provided a high frequency aesthetic device including: a high frequency transmitting part adapted to transmit the high frequency generated from an external high frequency generating source; and an aesthetic medicine injecting part adapted to apply the high frequency to a patient's skin and inject an aesthetic medicine into the skin, wherein the high frequency transmitting part and the aesthetic medicine injecting part are mechanically separable from each other.

According to the present invention, desirably, the high frequency transmitting part includes a casing in which a conductive member for high frequency application is accommodated and a connector adapted to be mechanically detachably coupled to the aesthetic medicine injecting part, and the aesthetic medicine injecting part includes a needle and a body adapted to apply the high frequency to the patient's skin and inject the aesthetic medicine into the skin.

According to the present invention, desirably, the needle has scales indicated on the insulation-coated outer peripheral surface thereof to allow the degree of insertion thereof to be checked quantitatively, and alternatively, the needle has different colors on the insulation-coated outer peripheral surface thereof to allow the degree of insertion thereof to be checked quantitatively.

According to the present invention, desirably, the needle has a blunt end portion.

According to the present invention, desirably, the casing further has an on/off switch adapted to electrically connect the high frequency transmitting part and the external high frequency generating source.

### Advantageous Effects of Invention

According to the present invention, there is provided the high frequency aesthetic device that is capable of separating the aesthetic medicine injecting part and the high frequency transmitting part from each other, thereby providing the convenience in use during the surgery, and that is capable of quantitatively checking the degree of insertion of the needle, thereby conducting the surgery in more accurate manner.

Additionally, the high frequency aesthetic device is capable of minimizing the damage of the blood vessels during the tunneling through the needle.

Moreover, while the high frequency aesthetic device according to the present invention is being applied for the aesthetic surgery, the frequency output levels or time can be freely selected by means of a surgeon, and the ways of inserting the needle can be effectively selected in accordance with the purposes of the surgery.

### Brief Description of Drawings

FIGS.1a and 1b are exemplary views showing conventional high frequency aesthetic devices.
FIG.2 is an exploded perspective view showing a high frequency aesthetic device according to the present invention, wherein an aesthetic medicine injecting part and a high frequency transmitting part are separated from each other.
FIG.3 is a perspective view showing the high frequency aesthetic device according to the present invention, wherein the aesthetic medicine injecting part and the high frequency transmitting part are coupled to each other.
FIG.4 is a side view showing the high frequency aesthetic device according to the present invention.
FIGS.5a and 5b are side views showing needles used in the high frequency aesthetic device according to the present invention.
FIG.6 is a side view showing the enlarged section of the needle of the high frequency aesthetic device according to the present invention.

### Mode for the Invention

The present invention should not be limited to the preferred embodiment described below, but may be modified in various forms without departing the spirit of the invention. Therefore, the various embodiments of the invention will be in detail explained with reference to the attached drawings. However, it should be understood that the invention is not limited to the preferred embodiment of the present invention, and many changes, variations and modifications of the constructional details illustrated and described may be resorted to without departing from the spirit of the invention.

In the description of the invention with reference to the attached drawings, further, the same components are indicated by the same reference numerals as each other, and for the brevity of the description, the explanation on their repeated features will be avoided. If it is determined that the detailed description on the known technology related to the invention makes the spirit of the invention unclear, it will be also avoided.

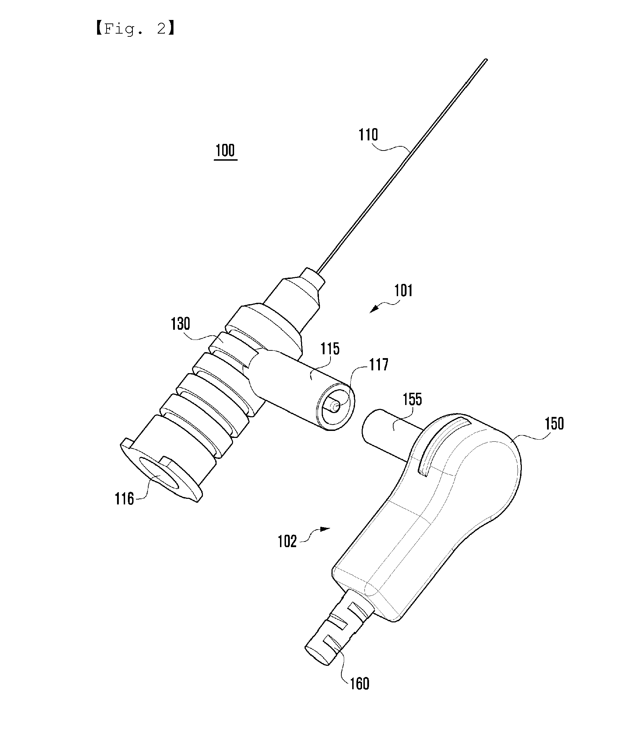

FIG.2 is an exploded perspective view showing a high frequency aesthetic device according to the present invention, wherein an aesthetic medicine injecting part and a high frequency transmitting part are separated from each other. As shown in FIG.2, a high frequency aesthetic device 100 according to the present invention largely includes an aesthetic medicine injecting part 101 and a high frequency transmitting part 102, which are mechanically separable from each other.

The high frequency transmitting part 102 is connected to a high frequency generating source (not shown) disposed at the outside and thus transmits the high frequency generated from the high frequency generating source. The high frequency transmitting part 102 includes a casing 150 in which a conductive member is accommodated and a connector 155. The casing 150 further includes an electrical connector mounted therein, which is adapted to electrically connect to the aesthetic medicine injecting part 101. The casing 150 serves to transmit the high frequency generated from the high frequency generating source through the conductive material for high frequency application generally having a shape of an electric wire to the aesthetic medicine injecting part 101. The connector 155 is disposed at one side of the casing 150 to allow the casing 150 to be mechanically detachably coupled to the aesthetic medicine injecting part 101. That is, the aesthetic medicine injecting part 101 and the high frequency transmitting part 102 are mechanically coupled to each other by means of the connector 155. If the aesthetic medicine injecting part 101 and the high frequency transmitting part 102 are mechanically coupled to each other, the high frequency generated from the high frequency generating source is transmitted from the electrical connector mounted inside the casing 150 through the conductive member accommodated in the casing 150 to the aesthetic medicine injecting part 101.

The aesthetic medicine injecting part 101 serves to apply the high frequency to a patient's skin and thus to inject an aesthetic medicine into the skin. The aesthetic medicine injecting part 101 largely includes a needle 110 and a body 130, and as shown in FIG.2, the needle 110 is passed through the body 130 in such a manner as to be protruded upwardly from the body 130. The body 130 includes a first coupling portion 116 adapted to be coupled to a container in which the aesthetic medicine is stored, a second coupling portion 117 adapted to be mechanically detachably coupled to the high frequency transmitting part 102, and an electrical connector 117 adapted to electrically connect the needle 110 and the high frequency transmitting part 102.

The needle 110 is made of an electrically conductive material and is electrically connected to the electrical connector 117. Accordingly, if the aesthetic medicine injecting part 101 and the high frequency transmitting part 102 are coupled to each other, the needle 110 receives the high frequency through the electrical connector 117 from the conductive member of the high frequency transmitting part 102. The high frequency is applied from the needle 110 to the skin of the patient through a surgeon, and then, the aesthetic medicine is injected into the skin of the patient. The detailed method for applying the aesthetic medicine to the skin of the patient will be explained below.

According to the present invention, the aesthetic medicine injection may be applied to every region needed, and generally, the aesthetic medicine may be injected into a nose, forehead, regions around zygomatic bone, nasolabial folds around a mouth, and so on. The aesthetic medicine has a main component such as, for example, hyaluronic acid, collagen, elastin, and so on, which is injected into the skin to provide volume and elasticity to the skin. According to the present invention, furthermore, the aesthetic medicine may become the fat obtained from the patient, which is used for fat graft.

At the time when the high frequency aesthetic device 100 according to the present invention is used, the needle 110 is inserted horizontally into the patient's skin. The needle 110 is inserted horizontally into the dermis layer of the skin, and it is moved up and down to perform tunneling. The tunneling allows a space in which the aesthetic medicine is accommodated to be formed into the dermis layer of the skin. Along the tunneled portion of the skin, after that, the high frequency is applied from the end of the needle 110. The high frequency stimulates the dermis layer to enable the tunneled portion into which the aesthetic medicine is injected to be firm. At this time, the up and down movements of the needle 110 means the starting point of the tunneling, and the application of the high frequency means the finishing point of the tunneling. Along the tunneled portion, next, the aesthetic medicine is injected from the needle 110.

The body 130 is empty at the inside thereof except for the needle 110 passed therethrough and is connected to the container in which the aesthetic medicine is stored through the first coupling portion 116, while being mechanically and electrically connected to the high frequency transmitting part 102 through the second coupling portion 115 and the electrical connector 117.

The first coupling portion 116 of the body 130 is formed on the underside of the body 130 and is physically coupled to the container in which the aesthetic medicine is stored. The aesthetic medicine is transmitted to the needle 110 through the first coupling portion 116 and is thus injected into the skin of the patient. The second coupling portion 115 of the body 130 is formed on the side of the body 130 and serves to allow the body 130 to be mechanically detachably coupled to the high frequency transmitting part 102. That is, if the second coupling portion 115 and the connector 155 are coupled to each other, the aesthetic medicine injecting part 101 is coupled to the high frequency transmitting part 102.

On the other hand, the electrical connector 117 of the body 130 accommodates a conductive member thereinto to electrically connect the needle 110 to the high frequency transmitting part 102. The conductive member of the electrical connector 117 is connected to the needle 110 passed through the interior of the body 130, and as mentioned above, accordingly, the high frequency is transmitted to the needle 110. That is, if the aesthetic medicine injecting part 101 is mechanically coupled to the high frequency transmitting part 102, the high frequency generated from the high frequency generating source is transmitted sequentially to the electrical connector disposed inside the casing 150, the electrical connector 117 of the body 130, and the needle 110. After that, the high frequency is applied to the inside of the skin from the needle 110 to enable the tunneled portion into which the aesthetic medicine is injected to be firm.

As shown in FIG.2, the high frequency aesthetic device 100 according to the present invention is capable of separating the aesthetic medicine injecting part 101 from the high frequency transmitting part 102. Such separation is needed because the application of the high frequency is not required anymore during the surgery process after the tunneling has been finished. That is, no high frequency is applied after the tunneled portion has been formed, and the aesthetic medicine is injected just along the tunneled portion. At this time, if the electric wire not needed is attached, it just hinders the operation of the surgeon. According to the present invention, the aesthetic medicine injecting part 101 is separable from the high frequency transmitting part 102, so that the operation of the surgeon can be easily conducted after the completion of the tunneling. Furthermore, such separable structure enables the cost for exchanging consumption goods to be reduced. That is, the aesthetic medicine injecting part 101 and the high frequency transmitting part 102 are exchangeable independently of each other, thereby decreasing their exchange cost.

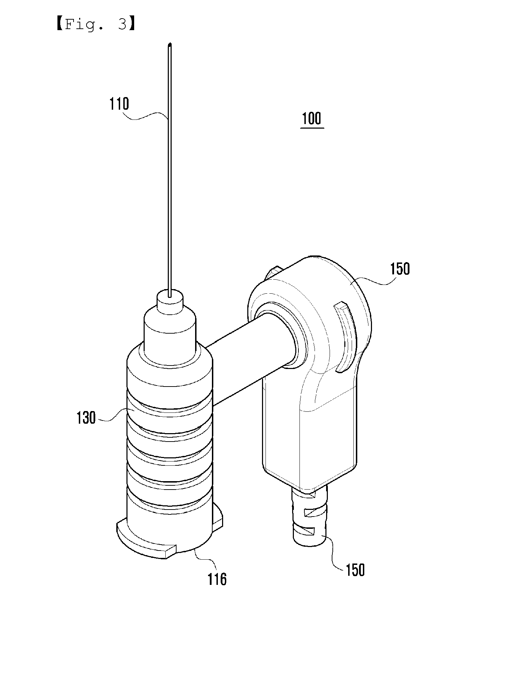

FIG.3 is a perspective view showing the high frequency aesthetic device according to the present invention, wherein the aesthetic medicine injecting part and the high frequency transmitting part are coupled to each other.

As shown in FIG.3, the connector 155 disposed on the side of the casing 150 is insertedly fitted to the second coupling portion 115 formed on the side of the body 130, thereby mechanically coupling the high frequency transmitting part 102 to the aesthetic medicine injecting part 101. As mentioned above, the needle 110 passed through the interior of the body 130 is connected to the electrical connector 117 through the conductive member, so that if the high frequency transmitting part 102 is coupled to the aesthetic medicine injecting part 101, the high frequency is transmitted to the needle 110. Accordingly, the high frequency generated from the high frequency generating source is transmitted to the needle 110 through the high frequency transmitting part 102.

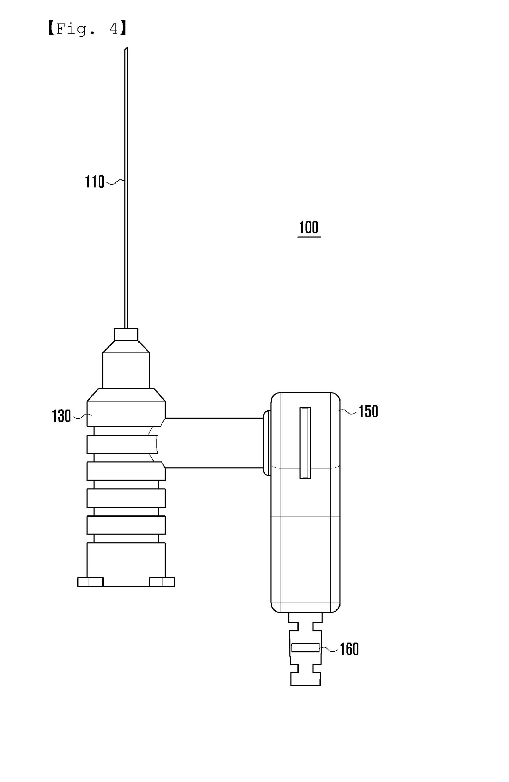

FIG.4 is a side view showing the high frequency aesthetic device according to the present invention.

Even though not shown in FIG.4, an on/off switch may be further provided on the high frequency transmitting part 102 of the high frequency aesthetic device 100 according to the present invention. The on/off switch serves to electrically connect the high frequency transmitting part 102 and the high frequency generating source. For example, if the high frequency transmitting part 102 and the aesthetic medicine injecting part 101 are mechanically separated from each other by means of the surgeon, the on/off switch is turned off to block the electrical connection therebetween. For example, the on/off switch may be mounted on the side of the casing 150.

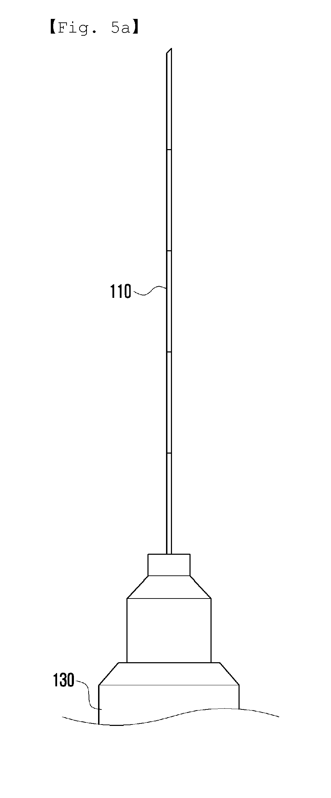

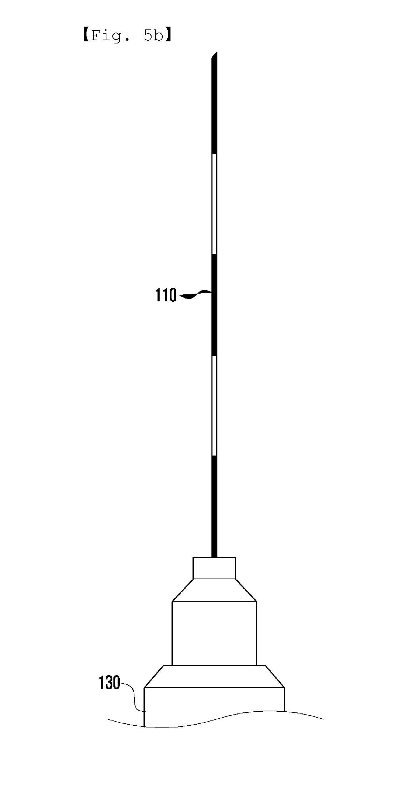

FIGS.5a and 5b are side views showing needles used in the high frequency aesthetic device according to the present invention.

The needle 110 is made of an electrically conductive material capable of applying high frequency to the interior of the skin, and in this case, the insulation coating is conducted on the outer peripheral surface of the needle 110, so that the high frequency is applied just from the end portion of the needle 110. In the preferred embodiment of the present invention, the insulation coating of the needle 110 is carried out with a polytetrafluoroetylene or silicon material.

As shown in FIG.5a, scales are indicated on the insulation-coated outer peripheral surface of the needle 110. So as to perform the application of the high frequency and the injection of the aesthetic medicine, the needle 110 should be inserted into the patient's skin, and in this case, the degree of insertion of the needle 110 can be checked quantitatively through the scales indicated on the needle 110.

As shown in FIG.5b, on the other hand, the insulation-coated outer peripheral surface of the needle 110 has different colors, which also allows the degree of insertion of the needle 110 to be checked quantitatively.

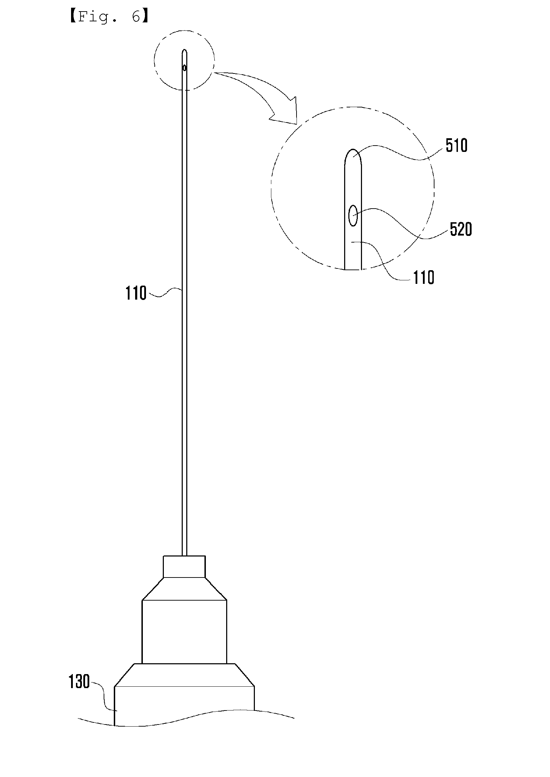

FIG.6 is a side view showing the enlarged section of the needle of the high frequency aesthetic device according to the present invention. Unlike general needles, the needle 110 used for the high frequency aesthetic device 100 according to the present invention has a blunt end portion 510.

If a general sharp end needle is used, it may damage the blood vessels around the tunneled portion during it is moved up and down to form the tunneled portion under the skin, thereby causing adverse effects like inflammation, edema, and so on. As shown in FIG.6, accordingly, if the end portion of the needle 110 is blunt, the damage of the blood vessels can be reduced, which remarkably removes the adverse effects like inflammation, edema, and so on.

First, the sharp end needle in the conventional practice is inserted horizontally into the dermis layer of the skin to form an insertion hole thereon, and next, the blunt end needle according to the present invention is inserted into the insertion hole to form the tunneled portion. After that, the high frequency is applied from the end portion 510 of the needle 110 where no insulation coating is applied, thereby finishing the tunneling, and the aesthetic medicine is injected through a hole 520 of the needle 110 as shown in FIG.6.

According to the preferred embodiment of the present invention, the high frequency aesthetic device is structured wherein the aesthetic medicine injecting part and the high frequency transmitting part are separable from each other and the degree of insertion of the needle can be checked quantitatively.

Additionally, the high frequency aesthetic device according to the present invention is capable of minimizing the damage of the blood vessels during the tunneling through the needle.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A high frequency aesthetic device comprising:
a high frequency transmitting part adapted to transmit the high frequency generated from an external high frequency generating source; and
an aesthetic medicine injecting part adapted to apply the high frequency to a patient's skin and inject an aesthetic medicine into the skin,
wherein the high frequency transmitting part and the aesthetic medicine injecting part are mechanically detachably coupled to each other.

2. The high frequency aesthetic device according to claim 1, wherein the aesthetic medicine injecting part comprises:
a needle made of an electrically conductive material and adapted to apply the high frequency to the skin of the patient and inject an aesthetic medicine into the skin; and
a body having a first coupling portion adapted to be coupled to a container in which the aesthetic medicine is stored, a second coupling portion adapted to be mechanically detachably coupled to the high frequency transmitting part, and an electrical connector adapted to electrically connect the needle and the high frequency transmitting part, the needle being passed through the body in such a manner as to be protruded upwardly from the body.

3. The high frequency aesthetic device according to claim 1, wherein the high frequency transmitting part comprises:
a casing in which a conductive member for high frequency application is accommodated, the conductive member being electrically connected to the external high frequency generating source, the casing having an electrical connector disposed at the inside thereof to conduct the electrical connection to the aesthetic medicine injecting part; and
a connector adapted to be mechanically detachably coupled to the aesthetic medicine injecting part.

4. The high frequency aesthetic device according to claim 2, wherein the needle has an insulation-coated outer peripheral surface thereof, and scales are indicated on the insulation-coated outer peripheral surface of the needle to allow the degree of insertion of the needle to be checked quantitatively.

5. The high frequency aesthetic device according to claim 2, wherein the needle has an insulation-coated outer peripheral surface thereof, and the colors of the insulation-coated outer peripheral surface of the needle are changed to allow the degree of insertion of the needle to be checked quantitatively.

6. The high frequency aesthetic device according to claim 2, 4 or 5, wherein the needle has a blunt end portion.

7. The high frequency aesthetic device according to claim 3, wherein the casing further comprises an on/off switch adapted to electrically connect the high frequency transmitting part and the external high frequency generating source.
